(19) Europäisches Patentamt / European Patent Office / Office européen des brevets

(11) **EP 2 184 300 B1**

(12) **EUROPEAN PATENT SPECIFICATION**

(45) Date of publication and mention of the grant of the patent:
**23.01.2019 Bulletin 2019/04**

(21) Application number: **08792469.2**

(22) Date of filing: **14.08.2008**

(51) Int Cl.:
*C08F 2/32* (2006.01)    *A61F 13/49* (2006.01)
*A61F 13/53* (2006.01)    *A61L 15/60* (2006.01)
*C08F 20/02* (2006.01)    *C08F 20/06* (2006.01)
*B01J 20/26* (2006.01)    *C08F 2/14* (2006.01)

(86) International application number:
**PCT/JP2008/064588**

(87) International publication number:
**WO 2009/025235 (26.02.2009 Gazette 2009/09)**

(54) **METHOD FOR PRODUCING WATER-ABSORBING RESIN SUITABLE FOR USE IN SANITARY PRODUCTS**

HERSTELLUNGSVERFAHREN FÜR WASSERABSORBIERENDES HARZ, DAS ZUR VEWENDUNG IN HYGIENEPRODUKTEN GEEIGNET IST

PROCÉDÉ DE PÉPARATION D'UNE RÉSINE ABSORBANT L'EAU APPROPRIÉE POUR ÊTRE UTILISÉE DANS DES PRODUITS SANITAIRES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.08.2007   JP 2007217173**

(43) Date of publication of application:
**12.05.2010   Bulletin 2010/19**

(73) Proprietor: **Sumitomo Seika Chemicals Co., Ltd.**
**Hyogo 675-0145 (JP)**

(72) Inventors:
• **YOKOYAMA, Hideki**
**Himeji-shi**
**Hyogo 672-8076 (JP)**

• **NAWATA, Yasuhiro**
**Kako-gun**
**Hyogo 675-0145 (JP)**

(74) Representative: **Mewburn Ellis LLP**
**City Tower**
**40 Basinghall Street**
**London EC2V 5DE (GB)**

(56) References cited:
EP-A1- 2 014 683    WO-A1-2004/101628
JP-A- H0 912 613    JP-A- H0 977 810
JP-A- H03 195 709    JP-A- H03 195 713
JP-A- 2006 068 731

Remarks:
The file contains technical information submitted after the application was filed and not included in this specification

**Description**

Technical Field

The present invention relates to a water-absorbent resin suitable for use in hygienic materials, and an absorbent material and an absorbent article using the same. More particularly, the present invention relates to a water-absorbent resin obtained by a reversed-phase suspension polymerization method, which contains a small amount of a petroleum hydrocarbon dispersion medium remaining therein used in reversed-phase suspension polymerization, and thus reducing an odor originating from the petroleum hydrocarbon dispersion medium, and also which is suitable for use in hygienic materials; an absorbent material and an absorbent article using the same.

Background Art

Water-absorbent resins are widely used in hygienic materials such as disposable diapers and sanitary napkins; daily commodities such as pet sheets; and industrial materials such as water blocking materials for cables.

Hygienic materials such as disposable diapers and sanitary napkins are generally composed of a top sheet, a back sheet, a hot melt adhesive, an elastic material, a water-absorbent resin and a pulp fiber, and use various synthetic resins and modifiers and, therefore, an odor originating from raw material components is felt from the hygienic materials, in some cases. Since these hygienic materials are worn on the human body, the odor makes those who wear them uncomfortable even if it is subtle and, therefore, it is desired to develop an odor-free material.

Among constituent materials of these hygienic materials, the water-absorbent resin has a subtle odor originating from the substances used in the production process, and since the odor tends to diffuse during absorbing water, it is considered to be desirable to reduce the odor.

Known water-absorbent resins used for the hygienic materials include, for example, a partially-neutralized product of polyacrylic acid, a neutralized product of a starch-acrylic acid graft polymer, a hydrolysate of a starch-acrylonitrile graft copolymer, a saponified product of a vinyl acetate-acrylic acid ester copolymer.

As the method for producing such the water-absorbent resin, an aqueous polymerization method and a reversed-phase suspension polymerization method are known, but in the case of the water-absorbent resin prepared by the reversed-phase suspension polymerization method in which polymerization is performed by suspending a water-soluble monomer in a dispersion medium, a major cause of the odor is considered to originate from the dispersion medium.

Known conventional arts for producing the water-absorbent resin by the reversed-phase suspension polymerization method include a method of polymerizing an aqueous solution of $\alpha,\beta$-unsaturated carboxylic acid and alkali metal salt thereof in a petroleum hydrocarbon solvent using a radical polymerization initiator in the presence or absence of a internal-crosslinking agent in which a sucrose fatty acid ester is used as a protective colloid agent (see Patent Document 1), and a method of polymerizing a 25% by mass or more of aqueous solution of an $\alpha,\beta$-unsaturated carboxylic acid and alkali metal salt thereof in a petroleum hydrocarbon solvent using a radical polymerization initiator in the presence or absence of a internal-crosslinking agent in which a polyglycerol fatty acid ester with an HLB of 2 to 16 is used as a surfactant (see Patent Document 2). However, these production technologies did not focus on reduction of an odor, and thus the resultant water-absorbent resins were not those with a low enough odor.

EP 2014683 A1 concerns a process for production of a water-absorbent resin by subjecting a water-soluble ethylenically unsaturated monomer to reversed-phase suspension polymerization, the process comprising the following steps (A) to (C): (A) adding an aqueous solution of water-soluble ethylenically unsaturated monomer to a petroleum hydrocarbon dispersion medium to disperse the aqueous solution in the dispersion medium; (B) adding a surfactant to the resultant dispersion to further disperse the aqueous solution, and (C) performing the reversed-phase suspension polymerization using a water-soluble radical polymerization initiator, optionally in the presence of an internal-crosslinking agent. JP 2006-068731 A concerns a particulate water-absorbing resin particles which polymerizes an unsaturated monomer containing an acid group and/or salt thereof and mainly comprises a water-absorbing resin particle which is at least 1 type in an nearly spherical shape, aggregates thereof, or aggregates derived from nearly spherical shape, and satisfies following (a), (b), (c), and (d): (a) centrifuge retention capacity (CRC) in a physiological saline is 32 or more g/g; (b) mass median particle diameter (D50) is not smaller than 200 $\mu$m and not larger than 400 pm; (c) ratio of particles having a particle diameter of smaller than 150 $\mu$m is not lower than 0 % by weight and not higher than 5 % by weight; and (d) content of volatile organic compounds as atmosphere concentration measured by a gas detector tube is not lower than 0 ppm and not higher than 100 ppm. JP 03-195713 A concerns a method for preparing a polymer having high water absorption properties wherein acrylic acid monomer which mainly comprises acrylic acid and(or) methacrylic acid and alkaline metal salt thereof or ammonium salt thereof is polymerised by a water-in-oil type reversed-phase suspension polymerisation in the presence of a water-soluble radical polymerisation initiator and hydroxyethyl cellulose by using a sorbitan fatty acid ester having HLB 3-6 as a dispersant, characterised in that the polymerisation of acrylic acid monomer is carried out in a cyclohexane solvent in the presence of a crosslinking agent. JP 03-195709 A concerns a method for preparing a polymer having high water absorption properties wherein acrylic acid monomer which mainly comprises acrylic acid and(or) methacrylic acid and alkaline metal salt thereof or ammonium salt thereof is polymerised by a water-in-oil type reversed-phase suspension polymerisation in which the acrylic acid monomer is sequentially supplied into a dispersion medium as the polymerisation progresses in the presence of a water-soluble radical polymerisation initiator by using a sorbitan fatty acid ester having HLB 3-12 as a dispersant, characterised in that the amount of the dispersant is 0.001 to 1 wt % based on the acrylic acid monomer and the polymerisation reaction is carried out under less than the reflux temperature.

Patent Document 1: JP-A No. 61-87702
Patent Document 2: JP-A No. 62-172006

Disclosure of the Invention

Problems to be Solved by the Invention

An object of the present invention is to provide a water-absorbent resin produced by a reversed-phase suspension polymerization method, which contains a small remaining amount of a petroleum hydrocarbon dispersion medium used in reversed-phase suspension polymerization, and thus reducing an odor originating from the petroleum hydrocarbon dispersion medium, when the water-absorbent resin absorbs water, and also which is suitable for use in hygienic materials; an absorbent material and an absorbent article.

Means for Solving the Problems

The present inventors intensively studied about a relation between an odor originating from a petroleum hydrocarbon dispersion medium, when the water-absorbent resin absorbs water, and a petroleum hydrocarbon dispersion medium used in production of the water-absorbent resin and, as a result, found out that a water-absorbent resin containing a specific amount or less of a petroleum hydrocarbon dispersion medium remaining therein is obtained by performing reversed-phase suspension polymerization at multi-stages of two or more stages in the production of the water-absorbent resin and adding a surfactant to a dispersion obtained after dispersing an aqueous solution of a water-soluble ethylenically unsaturated monomer in a petroleum hydrocarbon dispersion medium in reversed-phase suspension polymerization at the first stage, and thus significantly reducing an odor in the water-absorbent resin as compared with a water-absorbent resin obtained by the conventional art.

That is, the present invention relates to a method for producing a water-absorbent resin comprising performing a reversed-phase suspension polymerization at multi-stages of two or more stages, and performing the reversed-phase suspension polymerization at the first stage by:

(A) adding an aqueous solution of a water-soluble ethylenically unsaturated monomer to a petroleum hydrocarbon dispersion medium to disperse the aqueous solution in the dispersion medium,
(B) adding a surfactant to the resultant dispersion to further disperse the aqueous solution, and
(C) performing the reversed-phase suspension polymerization at the first stage using a water-soluble radical polymerization initiator, optionally in the presence of an internal-crosslinking agent

Effects of the Invention

According to the present invention, it is possible to provide a water-absorbent resin suitable for use in hygienic materials, which has a reduced odor originating from a petroleum hydrocarbon dispersion medium, when the water-absorbent resin absorbs water. An absorbent material and an absorbent article using the water-absorbent resin of the present invention are most suitable for use as hygienic materials because of less discomfort due to generation of an odor.

Best Mode for Carrying Out the Invention

The water-absorbent resin of the present invention is a water-absorbent resin obtained by subjecting an aqueous solution of a water-soluble ethylenically unsaturated monomer to reversed-phase suspension polymerization in a petroleum hydrocarbon dispersion medium in the presence of a surfactant, and the amount of the petroleum hydrocarbon dispersion medium remaining in the water-absorbent resin is 2,000 ppm or less.

The amount of the petroleum hydrocarbon dispersion medium remaining in the water-absorbent resin of the present invention is 2,000 ppm or less, preferably 1,500 ppm or less, more preferably 1,000 ppm or less, still more preferably 750 ppm or less, and even more preferably 500 ppm or less, in view of reducing an odor originating from a petroleum hydrocarbon dispersion medium, when the water-absorbent resin absorbs water. Although sensitivity to an odor varies depending on the kind of the petroleum hydrocarbon dispersion medium, when the amount of the remaining medium is 2,000 ppm or less, it is felt that an odor was significantly reduced as compared with a water-absorbent resin of the conventional art. The amount of the remaining medium is 500 ppm or less, it results in the level where little odor is felt.

In the present invention, the "amount of a remaining petroleum hydrocarbon dispersion medium" is the value measured by a measuring method described hereinafter.

As a conventional reversed-phase suspension polymerization method, a method of adding an aqueous monomer solution to a dispersion medium with a surfactant dissolved therein under stirring to disperse the aqueous monomer solution in the dispersion medium. However, regarding the water-absorbent resin obtained by these polymerization methods, an odor originating from the dispersion medium was felt, when the water-absorbent resin absorbs water.

In the production of a water-absorbent resin by a reversed-phase suspension polymerization method, a petroleum hydrocarbon having a boiling point of about 80 to 130°C is usually used as a dispersion medium. Therefore, it was found that, although it is considered that an odor originating from a dispersion medium is reduced by heating at the temperature of not less than the boiling point of the dispersion medium, heating actually has the low decreasing effect and little effect is exerted.

Thus, as a result of examination of the cause why an odor originating from a dispersion medium cannot be reduced by heating with respect to the water-absorbent resin obtained by a reversed-phase suspension polymerization method, it was found out that a small amount of the dispersion medium is confined in particles of the water-absorbent resin and an odor is felt by efflux of the dispersion medium, when the water-absorbent resin absorbs water.

In order to prevent the dispersion medium from confining and remaining in particles of the water-absorbent resin, the present inventors continued intensive study and found a method of effectively decreasing the amount of the remaining dispersion medium by dispersing an aqueous monomer solution to the dispersion medium and adding a surfactant to the resultant dispersion, followed by reversed-phase suspension polymerization.

That is, the method for obtaining the water-absorbent resin of the present invention is a method in which the reversed-phase suspension polymerization is performed at multi-stages of two or more stages, and the reversed-phase suspension polymerization at the first stage is performed by:

(A) adding an aqueous solution of a water-soluble ethylenically unsaturated monomer to a petroleum hydrocarbon dispersion medium to disperse the aqueous solution in the dispersion medium,
(B) adding a surfactant to the resultant dispersion to further disperse the aqueous solution, and
(C) performing the reversed-phase suspension polymerization at the first stage using a water-soluble radical polymerization initiator, optionally in the presence of an internal-crosslinking agent.

Examples of the water-soluble ethylenically unsaturated monomer used in the step (A) include monomers having an acid group, such as (meth)acrylic acid ["(meth)acrylic" means "acrylic" and "methacrylic", the same shall apply hereinafter] 2-(meth)acrylamide-2-methylpropanesulfonic acid and maleic acid, and salts thereof; nonionic unsaturated monomers such as (meth)acrylamide, N,N-dimethyl(meth)acrylamide, 2-hydroxyethyl (meth)acrylate and N-methylol(meth)acrylamide; and amino group-containing unsaturated monomers such as diethylaminoethyl (meth)acrylate and diethylaminopropyl (meth)acrylate, and quaternized monomers thereof. These water-soluble ethylenically unsaturated monomers can be used alone, or two or more kinds of them can be used in combination.

Among water-soluble ethylenically unsaturated monomers, (meth)acrylic acid and a salt thereof, and (meth)acrylamide are preferable in view of industrial availability.

When the water-soluble ethylenically unsaturated monomer has an acid group, it can also be used as a salt after neutralizing the acid group.

Examples of an alkaline compound used when a monomer having an acid group is converted into a salt by neutralization include compounds of lithium, sodium, potassium and ammonium.

When the monomer having an acid group is neutralized, the neutralization degree is preferably from 30 to 90 mol% of the acid group of the water-soluble ethylenically unsaturated monomer. When the neutralization degree is less than 30 mol%, the acid group is not easily ionized and water-absorption capacity decreases, and therefore it is not preferred. When the neutralization degree is more than 90 mol%, safety issues may arise when used as hygienic materials, and therefore it is not preferred.

The concentration of the monomer in the aqueous solution of the water-soluble ethylenically unsaturated monomer is from 20% by mass to saturation concentration.

If necessary, the aqueous solution of the water-soluble ethylenically unsaturated monomer may contain a chain transfer agent and a thickener.

Examples of the chain transfer agent include compounds such as thiols, thiolic acids, secondary alcohols, hypophosphorous acid and phosphorous acid. These chain transfer agents can be used alone, or two or more kinds of them can be used in combination.

Examples of the thickener include carboxymethyl cellulose, hydroxyethyl cellulose, hydroxypropyl cellulose, methyl cellulose, polyethylene glycol, polyacrylic acid, neutralized polyacrylic acid and polyacrylamide.

Examples of the petroleum hydrocarbon dispersion medium include aliphatic hydrocarbon having 6 to 8 carbon atoms, such as n-hexane, n-heptane, 2-methylhexane, 3-methylhexane and n-octane; alicyclic hydrocarbons having 6 to 8 carbon atoms, such as cyclohexane, methylcyclopentane and methylcyclohexane; and aromatic hydrocarbons such as benzene, toluene and xylene.

Among these petroleum hydrocarbon dispersion medium, at least one kind selected from an aliphatic hydrocarbon and an alicyclic hydrocarbone, each having 6 to 7 carbon atoms, or a mixture thereof is preferably used as the dispersion medium in view of industrial availability and the low cost.

The used amount of the petroleum hydrocarbon dispersion medium is usually from 50 to 600 parts by mass, more preferably from 50 to 400 parts by mass, and still more preferably from 50 to 200 parts by mass, based on 100 parts by mass of the aqueous solution of the water-soluble ethylenically unsaturated monomer in view of uniformly dispersing the aqueous solution of the water-soluble ethylenically unsaturated monomer and facilitating control of the polymerization temperature.

In the step (A), when the aqueous solution of the water-soluble ethylenically unsaturated monomer is added and dispersed in the petroleum hydrocarbon dispersion medium in the presence of a polymeric dispersion agent, it is effective to decrease the amount of the remaining petroleum hydrocarbon dispersion medium.

It is preferred to select and use, as the polymeric dispersion agent, those which are dissolved or dispersed in the petroleum hydrocarbon dispersion medium to be used, and examples of the polymeric dispersion agent include those having an average molecular weight of 20,000 or less, preferably 10,000 or less, and more preferably 5,000 or less. Specific examples thereof include maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene, an oxidized ethylene-propylene copolymer, an ethylene-acrylic acid copolymer, ethyl cellulose, ethylhydroxyethyl cellulose, anhydrous maleinated polybutadiene and anhydrous maleinated EPDM (ethylene/propylene/diene terpolymer).

Among these, at least one kind selected from the group consisting of maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene and an oxidized ethylene-propylene copolymer is preferred.

The additive amount of the polymeric dispersion agent is preferably 5 parts by mass or less, more preferably from 0.01 to 3 parts by mass, and still more preferably from 0.05 to 2 parts by mass, based on 100 parts by mass of the aqueous solution of the water-soluble ethylenically unsaturated monomer. When the additive amount of the polymeric dispersion agent is more than 5 parts by mass, it is not economic, being not preferable.

When the aqueous solution of the water-soluble ethylenically unsaturated monomer is added to and dispersed in the petroleum hydrocarbon dispersion medium, the aqueous solution of the water-soluble ethylenically unsaturated monomer is dispersed by stirring. However, stirring conditions vary depending on a desired dispersed droplet diameter and therefore cannot be determined unconditionally.

The dispersed droplet diameter can be adjusted by the kind of a stirring wing, the wing diameter and the rotation number.

It is possible to use, as the stirring impeller, a propeller impeller, a paddle impeller, an anchor impeller, a turbine impeller, a Pfaudler impeller, a ribbon impeller, a FULLZONE impeller (manufactured by Shinko Pantech Co., Ltd.), a MAXBLEND impeller (manufactured by Sumitomo Heavy Industries, Ltd.) and Super-Mix (manufactured by Satake Chemical Equipment Mfg., Ltd.).

A surfactant is added to the dispersion obtained in the step (A) and the aqueous solution of the water-soluble ethylenically unsaturated monomer is dispersed in the petroleum hydrocarbon dispersion medium (step (B)).

Examples of the surfactant used in the step (B) include nonionic surfactants such as sorbitan fatty acid ester, polyoxyethylene sorbitan fatty acid ester, polyglycerin fatty acid ester, polyoxyethylene glycerin fatty acid ester, sucrose fatty acid ester, sorbitol fatty acid ester, polyoxyethylene sorbitol fatty acid ester, polyoxyethylene alkyl ether, polyoxyethylene alkyl phenyl ether, polyoxyethylene castor oil, polyoxyethylene hardened castor oil, alkyl allyl formaldehyde condensed polyoxyethylene ether, polyoxyethylene polyoxypropyl alkyl ether, polyethylene glycol fatty acid ester, alkyl glucoside, N-alkyl gluconamide, polyoxyethylene fatty acid amide and polyoxyethylene alkylamine; and anionic surfactants such as fatty acid salt, alkylbenzene sulfonate, alkylmethyl taurate, polyoxyethylene alkyl phenyl ether sulfate, polyoxyethylene alkyl ether sulfate, polyoxyethylene alkyl ether sulfonic acid and a salt thereof, polyoxyethylene alkyl phenyl ether phosphoric acid and a salt thereof, and polyoxyethylene alkyl ether phosphoric acid and a salt thereof. These surfactants can be used alone, or two or more kinds of them can be used in combination.

Among these surfactants, at least one kind selected from the group consisting of polyglycerin fatty acid ester, sucrose fatty acid ester and sorbitan fatty acid ester are preferred in view of dispersion stability of the aqueous solution of the water-soluble ethylenically unsaturated monomer.

The additive amount of the surfactant used in the step (B) is preferably from 0.01 to 5 parts by mass, and more preferably from 0.05 to 3 parts by mass, based on 100 parts by mass of the aqueous solution of the water-soluble ethylenically unsaturated monomer. When the additive amount of the surfactant is less than 0.01 part by mass, dispersion stability of the aqueous monomer solution deteriorates, and therefore it is not preferred. When the additive amount of the surfactant is more than 5 parts by mass, it is not economic, being not preferable.

Although there is no particular limitation on the form of the surfactant added in the step (B), a method of using after diluting the surfactant with, or dissolving the surfactant in a small amount of the dispersion medium in advance is preferred since the surfactant is dispersed and stabilized within a short time.

In the step (B), in addition to the surfactant, the polymeric dispersion agent can be added.

The additive amount of the polymeric dispersion agent used in combination with the surfactant is preferably 5 parts by mass or less, more preferably from 0.01 to 3 parts by mass, and still more preferably from 0.05 to 2 parts by mass, based on 100 parts by mass of the aqueous solution of the water-soluble ethylenically unsaturated monomer. When the additive amount of the polymeric dispersion agent is more than 5 parts by mass, it is not economic, being not preferable. Since the polymeric dispersion agent to be added can be dispersed and stabilized within a short time, a method of using the polymeric dispersion agent in a state where the polymeric dispersion agent is dissolved or dispersed in a small amount of a dispersion medium while heating in advance is preferred.

The dispersion obtained in the step (B) is subjected to reversed-phase suspension polymerization, optionally in the presence of an internal-crosslinking agent using a water-soluble radical polymerization initiator to obtain a polymerization reaction solution (step (C)).

Examples of the water-soluble radical polymerization initiator include persulfates such as potassium persulfate, ammonium persulfate and sodium persulfate; peroxides such as hydrogen peroxide; and azo compounds such as 2,2'-azobis(2-amidinopropane) dihydrochloride, 2,2'-azobis[N-(2-carboxyethyl)-2-methylpropiondiamine] tetrahydrate, 2,2'-azobis(1-imino-1-pyrrolidino-2-methylpropane) dihydrochloride and 2,2'-azobis[2-methyl-N-(2-hydroxyethyl)-propionamide].

Among these water-soluble radical polymerization initiators, potassium persulfate, ammonium persulfate, sodium persulfate and 2,2'-azobis(2-amidinopropane) dihydrochloride are preferred in view of availability and ease of handling.

It is also possible to use, as a redox polymerization initiator, the water-soluble radical polymerization initiator in combination with reducing agents such as sulfite and ascorbic acid.

The used amount of the water-soluble radical polymerization initiator is usually from 0.01 to 1 part by mass based on 100 parts by mass of the water-soluble ethylenically unsaturated monomer. When the amount is less than 0.01 part by mass, a polymerization rate decreases, whereas, when the amount is more than 1 part by mass, a rapid polymerization reaction arises. Therefore, both cases are not preferred.

Although there is no particular limitation on timing of the addition of the water-soluble radical polymerization initiator, it is preferred to add the water-soluble radical polymerization initiator to the aqueous solution of the water-soluble ethylenically unsaturated monomer in advance.

Examples of the internal-crosslinking agent used optionally include polyols such as (poly)ethylene glycol ["(poly)" means the case where a prefix "poly" exists or not, the same shall apply hereinafter], 1,4-butanediol, glycerin and trimethylolpropane; polyunsaturated esters having two or more vinyl groups obtained by reacting polyols with an unsaturated acid such as acrylic acid or methacrylic acid; bisacrylamides such as N,N'-methylenebisacrylamide; and polyglycidyl compounds having two or more glycidyl groups, such as (poly)ethylene glycol diglycidyl ether, (poly)ethylene glycol triglycidyl ether, (poly)glycerin diglycidyl ether, (poly)glycerin triglycidyl ether, (poly)propylene glycol polyglycidyl ether and (poly)glycerol polyglycidyl ether. These internal-crosslinking agents can be used alone, or two or more kinds of them can be used in combination.

The additive amount of the internal-crosslinking agent is preferably 3 parts by mass or less, more preferably 1 part by mass or less, and still more preferably from 0.001 to 0.1 part by mass, based on 100 parts by mass of the water-soluble ethylenically unsaturated monomer. When the amount is more than 3 parts by mass, excess crosslinking arises and water-absorption capability excessively deteriorates, and therefore it is not preferred.

It is preferred that the internal-crosslinking agent is added to the aqueous solution of the water-soluble ethylenically unsaturated monomer in advance.

The reaction temperature during reversed-phase suspension polymerization in the present invention varies depending on the kind and amount of the polymerization initiator to be used, and therefore cannot be determined unconditionally, but is preferably from 20 to 100°C, and more preferably from 40 to 90°C. When the reaction temperature is lower than 20°C, the polymerization rate may decrease, whereas, when the reaction temperature is higher than 100°C, a rapid polymerization reaction arises. Therefore, both cases are not preferred.

The size of particles obtained by the polymerization of the water-soluble ethylenically unsaturated monomer at the first stage is preferably from 20 to 200 μm, more preferably from 30 to 150 μm, and still more preferably from 40 to 100 μm, in terms of a median particle size in view of obtaining a proper aggregated particle size at multi-stage polymerization. The median particle size of the first-stage polymerized particles is the measured value of particles obtained by dehydration and drying after completion of the polymerization at the first stage.

To the polymerization reaction suspension obtained after completion of the step (C), the aqueous solution of the water-soluble ethylenically unsaturated monomer is added and, subsequently, reversed-phase suspension polymerization at the second stage is performed.

An aqueous solution of a water-soluble ethylenically unsaturated monomer at the second stage is added so as to aggregated particles obtained by the polymerization at the first stage thereby adjusting the particle size suitable for use in hygienic materials.

Therefore, it is necessary to decrease the function of the surfactant so as not to form independent droplets by the aqueous solution of a water-soluble ethylenically unsaturated monomer at the second stage. For example, the aggregated particles can be obtained by cooling after completion of the polymerization at the first stage and adding a water-soluble ethylenically unsaturated monomer at the second stage at the temperature at which the surfactant is precipitated.

The method is not limited to the above method as long as it is a method capable of obtaining aggregated particles by the addition of the water-soluble ethylenically unsaturated monomer at the second stage.

The amount of the remaining dispersion medium can be further decreased by performing reversed-phase suspension polymerization at the second stage.

It is possible to use, as water-soluble ethylenically unsaturated monomer at the second-stage, the same as those exemplified as the water-soluble ethylenically unsaturated monomer at the first stage. The kind, the neutralization degree and the neutralized salt of the monomer, and the concentration of the aqueous monomer solution may be the same as or different from those of the water-soluble ethylenically unsaturated monomer at the first stage.

The polymerization initiator to be added to an aqueous solution of a water-soluble ethylenically unsaturated monomer at the second stage can also be used after appropriately selecting from those exemplified as the polymerization initiator used in the polymerization at the first stage.

If necessary, an internal-crosslinking agent and a chain transfer agent can also be added to the aqueous solution of the water-soluble ethylenically unsaturated monomer at the second stage, and can be used after selecting from those exemplified during the polymerization at the first stage.

The additive amount of the water-soluble ethylenically unsaturated monomer at the second stage is preferably from 50 to 300 parts by mass, more preferably form 100 to 200 parts by mass, and still more preferably from 120 to 160 parts by mass, based on 100 parts by mass of the water-soluble ethylenically unsaturated monomer at the first stage in view of obtaining appropriate aggregated particles.

It is sufficient that the entire components are mixed uniformly by stirring in the reversed-phase suspension polymerization at the second stage. The median particle size of aggregated particles can be controlled depending on the precipitated state of the surfactant and the ratio of the amount of the ethylenically unsaturated monomer at the second stage to the ethylenically unsaturated monomer at the first stage.

The median particle size of the aggregated particles suitable for use in hygienic materials is preferably from 200 to 600 μm, more preferably from 250 to 500 μm, and still more preferably from 300 to 450 μm.

Although the reaction temperature in reversed-phase suspension polymerization at the second stage varies depending on the kind and amount of the polymerization initiator and therefore cannot be determined unconditionally, but is preferably from 20 to 100°C, and more preferably from 40 to 90°C.

Furthermore, for the purpose of improving productivity, multi-stage reversed-phase suspension polymerization can be performed by performing the polymerization reaction at the third stage or subsequent stage similar to reversed-phase suspension polymerization at the second stage.

After completion of the reversed-phase suspension polymerization at multi-stages, it is preferred to add a post-crosslinking agent containing two or more functional groups having reactivity with a functional group derived from water-soluble ethylenically unsaturated monomer to the resultant precursor of the water-absorbent resin. By a reaction by adding the post-crosslinking agent after the polymerization, the crosslinking density of the surface layer of water-absorbent resin particles increases and various performances such as water-absorption capacity under load, water-absorption rate and gel strength can be enhanced, and thus imparting performances suitable for use in hygienic materials.

A post-crosslinking agent to be used in the post-crosslinking reaction is not particularly limited as long as it can react with a functional group derived from the water-soluble ethylenically unsaturated monomer used in the polymerization.

Examples of the post-crosslinking agent to be used include polyols such as ethylene glycol, propylene glycol, 1,4-butanediol, trimethylolpropane, glycerin, polyoxyethylene glycol, polyoxypropylene glycol and polyglycerin; polyglycidyl compounds such as (poly)ethylene glycol diglycidyl ether, (poly)ethylene glycol triglycidyl ether, (poly)glycerin diglycidyl ether, (poly)glycerin triglycidyl ether, (poly)propylene glycol polyglycidyl ether and (poly)glycerol polyglycidyl ether; haloepoxy compounds such as epichlorohydrin, epibromohydrin and α-methylepichlorohydrin; compound having two or more reactive functional groups, for example, isocyanate compounds such as 2,4-tolylene diisocyanate and hexamethylene diisocyanate; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol and 3-butyl-3-oxetane ethanol; oxazoline compounds such as 1,2-ethylenebisoxazoline; and carbonate compounds such as ethylene carbonate. These post-crosslinking agents can be used alone, or two or more kinds of them can be used in combination.

Among these, polyglycidyl compounds such as (poly)ethylene glycol diglycidyl ether, (poly)ethylene glycol triglycidyl ether, (poly)glycerin diglycidyl ether, (poly)glycerin triglycidyl ether, (poly)propylene glycol polyglycidyl ether and (poly)glycerol polyglycidyl ether are preferred in view of excellent reactivity.

The additive amount of the post-crosslinking agent is preferably from 0.01 to 5 parts by mass, and more preferably from 0.02 to 3 parts by mass, based on 100 parts by mass of the total amount of the water-soluble ethylenically unsaturated monomer subjected to the polymerization.

When the additive amount of the post-crosslinking agent is less than 0.01 part by mass, it is impossible to enhance various performances such as water-absorption capacity under load, water-absorption rate and gel strength of the resultant water-absorbent resin, whereas, when the additive amount is more than 5 parts by mass, water-absorption capacity excessively deteriorates, and therefore both cases are not preferred.

The post-crosslinking agent may be added as it is, or added in the form of an aqueous solution. If necessary, the post-crosslinking agent may be added using a hydrophilic organic solvent as a solvent. Examples of the hydrophilic organic solvent include lower alcohols such as methyl alcohol, ethyl alcohol, n-propyl alcohol, isopropyl alcohol and propylene glycol; ketones such as acetone and methyl ethyl ketone; ethers such as diethyl ether, dioxane and tetrahydrofuran; amides such as N,N-dimethylformamide; and sulfoxides such as dimethyl sulfoxide. These hydrophilic organic solvents can be used alone, or two or more kinds of them can be used in combination.

The timing of the addition of the post-crosslinking agent may be after completion of the polymerization and is not particularly limited. The post-crosslinking reaction is preferably performed in a drying step after the polymerization in the presence of water at the amount within a range from 1 to 200 parts by mass, more preferably from 5 to 100 parts by mass, and still more preferably from 10 to 50 parts by mass, based on 100 parts by mass of the water-absorbent resin. By adjusting the amount of water during the addition of the post-crosslinking agent, post-crosslinking in the surface layer of particles of the water-absorbent resin can be more suitably performed and excellent water-absorption capability can be exhibited.

The temperature in the post-crosslinking reaction is preferably from 50 to 250°C, more preferably from 60 to 180°C, still more preferably from 60 to 140°C, and even more preferably from 70 to 120°C.

In the present invention, the drying step can be performed under a normal pressure or reduced pressure, or can be performed under a gas flow such as nitrogen gas flow in order to enhance drying efficacy. When the drying step is performed under a normal pressure, the drying temperature is preferably from 70 to 250°C, more preferably from 80 to 180°C, still more preferably from 80 to 140°C, and even more preferably from 90 to 130°C. When the drying step is performed under reduced pressure, the drying temperature is preferably from 60 to 100°C, and more preferably from 70 to 90°C.

The water content of the water-absorbent resin after drying is 20% or less, and preferably 10% or less, in view of imparting fluidity. An amorphous silica powder can also be added to the water-absorbent resin so as to improve fluidity.

The absorbent material using the water-absorbent resin of the present invention is composed of particles of a water-absorbent resin and a hydrophilic fiber. Examples of the constitution of the absorbent material include a mixed structure in which particles of a water-absorbent resin and a hydrophilic fiber are uniformly blended, a sandwich structure in which particles of a water-absorbent resin are held between layered hydrophilic fibers, and a structure in which particles of a water-absorbent resin and a hydrophilic fiber are wrapped with a tissue, but the present invention is not limited thereto. The absorbent material can contain a synthetic fiber as a reinforcing agent.

The content of the water-absorbent resin in the absorbent material is preferably from 5 to 80% by mass, and more preferably from 15 to 60% by mass. When the content of the water-absorbent resin is less than 5% by mass, absorption capacity may decrease, leading to increase in liquid leakage and re-wet. When the content of the water-absorbent resin is more than 80% by mass, the cost of the absorbent material may increase and the touch of the absorbent material may become hard.

Examples of the hydrophilic fiber include cellulose fibers such as cotton-like pulp obtained from wood, mechanical pulp, chemical pulp and semichemical pulp; and artificial cellulose fibers made of rayon and acetate, but the present invention is not limited only thereto. The hydrophilic fiber can contain a fiber made of a synthetic resin such as polyamide, polyester or polyolefin.

The absorbent article using the water-absorbent resin of the present invention has a structure of holding the absorbent material between a liquid-permeable sheet (top sheet) through which an aqueous liquid can permeate and a liquid-impermeable sheet (back sheet) through which an aqueous liquid can not permeate. The liquid-permeable sheet is disposed on the side which is contacted with the body, while the liquid-impermeable sheet is disposed on the side which is not contacted with the body.

Examples of the liquid-permeable sheet include a non-woven fabric made of polyethylene, polypropylene, polyester or polyamide, and a porous synthetic resin sheet. Examples of the liquid-impermeable sheet include a film made of polyethylene, polypropylene, polyester or polyamide, and a film made of a composite material of these synthetic resins and a non-woven fabric, but the present invention is not limited only thereto.

The size of the liquid-permeable sheet and the liquid-impermeable sheet varies depending on applications of the absorbent article and therefore cannot be determined unconditionally. Therefore, it is preferred that the size is appropriately adjusted according to the applications.

Examples

The present invention will be described in detail by way of Examples, but the present invention is not limited only to these Examples.

The median particle size, the water content, the absorption capacity of physiological saline solution, the amount of the remaining dispersion medium (amount of petroleum hydrocarbon dispersion medium remaining in water-absorbent resin particles) and the odor sensory test of water-absorbent resins obtained in the respective Examples and Comparative Examples were evaluated by the following methods.

(1) Median particle size

A water-absorbent resin(50g) was passed through a JIS standard sieve having a sieve opening size of 250 $\mu$m. The median particle size was measured using a combination of sieves (A) when 50% by mass or more of the resin passed through the sieve, while using a combination of sieves (B) when 50% by mass or more of the resin remaining on the sieve.

(A) JIS standard sieves were combined in a downward order of; a sieve having a sieve opening size of 425 $\mu$m at the top, a sieve having a sieve opening of 250 $\mu$m, a sieve having a sieve opening size of 180 $\mu$m, a sieve having a sieve opening size of 150 $\mu$m, a sieve with a sieve opening size of 106 $\mu$m, a sieve with a sieve opening size of 75 $\mu$m, a sieve having a sieve opening size of 45 $\mu$m and a tray.

(B) JIS standard sieves were combined in a downward order of; a sieve having a sieve opening size of 850 $\mu$m at the top, followed by a sieve having a sieve opening size of 600 $\mu$m, a sieve having a sieve opening size of 500 $\mu$m, a sieve having a sieve opening size of 425 $\mu$m, a sieve having a sieve opening size of 300 $\mu$m, a sieve having a sieve opening size of 250 $\mu$m, a sieve having a sieve opening size of 150 $\mu$m and a tray.

About 50 g of the water-absorbent resin was placed on the uppermost sieve of the combination, and classified for 20 minutes using a Rotap-type shaking machine.

After the sieve classification, the mass of the water-absorbent resin remaining on the respective sieves was calculated in terms of mass% based on the total mass of resin, the values were integrated in an order from the resins with a larger particle size, and thereby the relations between the sieve openings and integration values of the mass% of the water-absorbent resin remaining on the sieve were plotted on a logarithmic-probability paper. The plots on the logarithmic-probability paper were connected with a straight line, and the particle size corresponding to integrated mass% of 50% by mass was defined as the median particle size.

(2) Water content

About 2.5 g of the water-absorbent resin was accurately weighed (X g) on an aluminum cup, and after drying at 105°C with a hot air drier for 2 hours, the mass of the dried water-absorbent resin was measured (Y g), and then the water content was calculated by the following equation (it is assumed that tare mass of an aluminum cup does not change before and after drying).

$$\text{Water content (\%)} = (X - Y)/X \times 100$$

(3) Absorption capacity of physiological saline solution

500 g of a 0.9 mass% aqueous solution of sodium chloride was placed into a 500 mL beaker with a 3 cm-long magnetic stirrer bar and, while stirring the solution with a magnetic stirrer, 2.0 g of the water-absorbent resin was accurately weighed (A g) and added to the beaker such that no unswollen lump was left, and then the mixture was stirred at a rate of 600 rpm for 1 hour.

A gel was filtered out by a JIS standard sieve which is 20 cm in diameter with a sieve opening of 106 $\mu$m, excessive water contained in the gel remaining on the sieve was roughly drained with a fluorine resin board, and then the sieve was tilted and left for 30 minutes for further draining. The mass of the gel remaining on the sieve (B g) was weighed, and the absorption capacity of physiological saline solution was calculated by the following equation.

Absorption capacity of physiological saline solution

$$(g/g) = B/A$$

(4) Amount of remaining dispersion medium

The amount of the petroleum hydrocarbon dispersion medium remaining in the water-absorbent resin was measured using a head-space gas chromatograph.

(a) Formation of calibration curve

0.1 g of the petroleum hydrocarbon dispersion medium (hereinafter referred to as a "dispersion medium") used to polymerize the sample was accurately weighed into a 50 ml volumetric screw vial and then DMF (dimethylformamide) was added thereto to accurately make 40 g, followed by stirring with a magnetic stirrer bar to obtain a standard sample solution.

In a 20 ml volumetric vial bottle (No. 5, manufactured by Maruemu Corporation), each of 0.01, 0.04, 0.2 and 0.5 g of the standard sample solutions was accurately weighed and DMF was added thereto to make the amount of contents in each vial bottle to 0.75 g. Furthermore, 0.75 g of distilled water was added to each vial, followed by stopping with a septum rubber and an aluminum cap and further fastening.

This vial bottle was warmed at 110°C for 2 hours, and 1 ml of a vapor phase portion was collected and then injected into a gas chromatograph to obtain a chromatogram.

A calibration curve was made from a charge amount of a dispersion medium in each vial bottle and a peak area of the chromatogram (when a mixture of petroleum hydrocarbons was used as the dispersion medium, plural peaks appeared and therefore a calibration curve was made from a total value of the areas and the charge amount).

(b) Measurement of amount of dispersion medium remaining in sample

About 2 g of a sample to be measured was charged into an aluminum cup and then dried with a hot air dryer at 105°C for 2 hours to adjust the water content.

Into a 20 ml volumetric vial bottle (No.5, manufactured by Maruemu Corporation), 0.10 g of the above sample was accurately weighed and 0.75 g of DMF was added, and also 0.75 g of distilled water was added. After shaking the bottle and slightly stirring, the vial was stopped with a septum rubber and an aluminum cap, followed by fastening (By adding distilled water in the presence of DMF, a water-absorbent resin was swollen slowly and uniformly and the included dispersion medium was extracted and detected).

This vial bottle was warmed at 110°C for 2 hours, and 1 ml of a vapor phase portion was collected and then injected into a gas chromatograph to obtain a chromatogram.

The amount of the dispersion medium contained in 0.10 g of the sample was calculated from the calibration curve made based on the peak area of the resultant chromatogram, and then converted into the amount [ppm] of the dispersion medium contained per 1 g of the sample.

The conditions of a gas chromatograph used in the measurement of the amount of the remaining dispersion medium in the present invention are as follows. Model: GC-14A + HSS2B (HEADSPACE Autosampler) manufactured by Shimadzu Corporation

Filler: Squalane 25% Shimalite (NAW) (101)

80-100 mesh

Column: 3.2 mm$\varphi$ $\times$ 2 m

Column temperature: 80°C

Injection port temperature: 180°C

Detector temperature: 180°C

Detector: FID

Gas carrier: N$_2$

Vial bottle heating temperature: 110°C
Syringe setting temperature: 110°C

(5) Odor sensory test

An odor of the water-absorbent resin originating from the dispersion medium upon swelling was compared by the following method. To a 140 mL volumetric glass bottle with a lid (mayonnaise bottle), 20.0 g of 0.9% by mass saline at 25°C was charged, and stirred with a 3 cm-long magnetic stirrer bar. To the glass bottle, 4.0 g of the water-absorbent resin was added and the bottle was tightly sealed. The odor originating from the dispersion medium in the glass bottle was determined by five panelists in accordance with the "six-level odor intensity indication method" shown below, and evaluated by the average.

Table 1

| Six-level evaluation | Evaluation criteria |
| --- | --- |
| 5 | Very strong odor |
| 4 | Strong odor |
| 3 | Easily recognizable odor |
| 2 | Recognizable slight odor |
| 1 | Barely sensed odor |
| 0 | No odor |

Example 1

Into a 500 mL Erlenmeyer flask, 92.0 g of 80% by mass of acrylic acid was charged and neutralized by adding dropwise 102.2 g of 30 mass% sodium hydroxide under stirring while cooling from the outside. To this were added 0.11 g of potassium persulfate, 8.3 mg of ethylene glycol diglycidyl ether and 43.6 g of ion-exchange water to prepare an aqueous solution of a water-soluble ethylenically unsaturated monomer (At this time, the aqueous monomer solution had a neutralization degree of 75 mol% and a concentration of 38% by mass).
To a 2 L volumetric five-necked cylindrical round-bottom flask equipped with a stirrer with two 50 mmϕ pitched blade paddle impellers, a thermometer, a reflux condenser and a nitrogen gas introducing tube, 334 g of n-heptane was added as a petroleum hydrocarbon dispersion medium and this was warmed to 61°C, and then the aqueous solution of the water-soluble ethylenically unsaturated monomer was added at once under a stirring rate of 500 rpm using a funnel and dispersed by stirring at an inner temperature of 40°C for 10 minutes (step (A))
Next, a solution prepared by warming 0.92 g of a sucrose fatty acid ester (manufactured by Mitsubishi-Kagaku Foods Corporation, trade name: S-370) as a surfactant to dissolve it in 8.28 g of n-heptane by warming was added to the round-bottom flask using a funnel and an aqueous solution of a water-soluble ethylenically unsaturated monomer was further dispersed (step (B))
Next, the atmosphere in the system was well substituted with nitrogen while maintaining an inner temperature of the round-bottom flask containing the dispersion at 40°C and the polymerization reaction was performed by warming for 1 hour using a hot water bath at 70°C (step (C)).
After completion of the polymerization at the first stage, the stirring rate was increased to 1,000 rpm and the inner temperature was lowered to about 21°C.
Separately, to a 500 mL Erlenmeyer flask, 128.8 g of 80 mass% acrylic acid was added and neutralized by adding dropwise 142.9 g of 30 mass% sodium hydroxide under stirring while cooling from the outside. To this were added 0.15 g of potassium persulfate, 11.6 mg of ethylene glycol diglycidyl ether and 16.7 g of distilled water to prepare an aqueous solution of a water-soluble ethylenically unsaturated monomer at the second stage (At this time, the aqueous solution of the monomer solution at the second stage had a neutralization degree of 75 mol% and a concentration of 44% by mass).
Next, the aqueous monomer solution at the second stage was added to the cooled polymerization suspension at the first stage using a dropping funnel. After the atmosphere in the system was well substituted with nitrogen, reversed-phase suspension polymerization at the second stage was performed by warming for 1 hour using a hot water bath at 70°C.
After the polymerization reaction at the second stage, the reaction suspension was heated using an oil bath at 120°C and about 260 g of water was removed off the system by azeotropic distillation while refluxing n-heptane in the flask to obtain a dehydrated polymer dispersed in heptane. To the resultant heptane dispersed dehydrated polymer, 8.2 g of a 2% aqueous solution of ethylene glycol diglycidyl ether as a post-crosslinking agent was added and the post-crosslinking reaction was performed at 83°C for 2 hours.

After heating using an oil bath at 120°C, n-heptane and water were removed off the system by distillation, followed by drying under a nitrogen gas flow to obtain 234 g of a water-absorbent resin having the shape of aggregated spherical particles. This water-absorbent resin had a median particle size of 310 $\mu$m, absorption capacity of physiological saline solution of 63 g/g, and a water content of 4.5% (A median particle size of primary particles is about 60 $\mu$m).

Example 2

According to the same manner as that of Example 1, except that a solution prepared by warming 0.92 g of a maleic anhydride-modified ethylene-propylene copolymer (manufactured by Mitsui Chemicals, Inc., trade name: HIWAX 1105A) to dissolve it in 8.28 g of n-heptane by warming was added as a polymeric dispersion agent after adding a solution of a surfactant in the step (B) of Example 1, 236 g of a water-absorbent resin having the shape of aggregated spherical particles was obtained. This water-absorbent resin had a median particle size of 403 $\mu$m, absorption capacity of physiological saline solution of 65 g/g, and a water content of 3.7% (A median particle size of primary particles of this water-absorbent resin is about 60 $\mu$m).

Example 3

To a 500 mL Erlenmeyer flask, 92.0 g of 80 mass% acrylic acid was added and this was neutralized by adding dropwise 102.2 g of 30 mass% sodium hydroxide while cooling the flask from the outside. To this were added 0.11 g of potassium persulfate, 8.3 mg of ethylene glycol diglycidyl ether and 43.6 g of ion-exchange water to prepare an aqueous solution of a water-soluble ethylenically unsaturated monomer.

To a 2 L volumetric five-necked cylindrical round-bottom flask equipped with a stirrer with two 50 mm$\phi$ pitched blade paddle impellers, a thermometer, a reflux condenser and a nitrogen gas introducing tube, 334 g of n-heptane was added as a petroleum hydrocarbon dispersion medium and 0.92 g of a maleic anhydride-modified ethylene-propylene copolymer (manufactured by Mitsui Chemicals, Inc., trade name: HIWAX 1105A) was added as a polymeric dispersion agent and then this was warmed to dissolve it. After cooling to 61°C, the aqueous solution of a water-soluble ethylenically unsaturated monomer was added at once under a stirring rate of 500 rpm using a funnel and then dispersed by stirring at an inner temperature of 40°C for 10 minutes (step (A))

Next, a solution prepared by warming 0.92 g of a sucrose fatty acid ester (manufactured by Mitsubishi-Kagaku Foods Corporation, trade name: S-370) as a surfactant to dissolve it in 8.28 g of n-heptane by warming was added to the round-bottom flask using a funnel and an aqueous solution of a water-soluble ethylenically unsaturated monomer was further dispersed (step (B))

Next, the atmosphere in the system was well substituted with nitrogen while maintaining the inner temperature of the round-bottom flask containing the dispersion at 40°C and the polymerization reaction was performed by warming for 1 hour using a hot water bath at 70°C (step (C)).

After completion of the polymerization at the first stage, the stirring rate was increased to 1,000 rpm and the inner temperature was lowered to about 23°C.

Separately, to a 500 mL Erlenmeyer flask, 128.8 g of 80 mass% acrylic acid was added and neutralized by adding dropwise 142.9 g of 30 mass% sodium hydroxide under stirring while cooling from the outside. To this were added 0.15 g of potassium persulfate, 11.6 mg of ethylene glycol diglycidyl ether and 16.7 g of distilled water to prepare an aqueous solution of a water-soluble ethylenically unsaturated monomer at the second stage. Next, the aqueous monomer solution at the second stage was added to the cooled polymerization suspension using a dropping funnel. After the atmosphere in the system was well substituted with nitrogen, reversed-phase suspension polymerization at the second stage was performed by warming for 1 hour using a hot water bath at 70°C.

After the polymerization reaction at the second stage, the reaction solution was heated using an oil bath at 120°C and about 260 g of water was removed off the system by azeotropic distillation while refluxing n-heptane in the flask to obtain a dehydrated polymer dispersed in heptane. To the resultant heptane dispersed dehydrated polymer, 8.2 g of a 2% aqueous solution of ethylene glycol diglycidyl ether as a post-crosslinking agent was added and the post-crosslinking reaction was performed at 83°C for 2 hours.

After heating using an oil bath at 120°C, n-heptane and water were removed off the system by distillation, followed by drying under a nitrogen gas flow to obtain 238 g of a water-absorbent resin having the shape of aggregated spherical particles. This water-absorbent resin had a median particle size of 352 $\mu$m, absorption capacity of physiological saline solution of 62 g/g, and a water content of 5.6% (A median particle size of primary particles of this water-absorbent resin is about 60 $\mu$m).

Example 4

According to the same manner as that of Example 3, except that a solution prepared by warming to dissolve the surfactant and, then, 0.92 g of an oxidized ethylene-propylene copolymer (manufactured by Mitsui Chemicals, Inc., trade name: HIWAX 220MP) as a polymeric dispersion agent in 8.28 g of n-heptane by warming was added in the step (B) of Example 3, 237 g of a water-absorbent resin having the shape of aggregated spherical particles spherical particles was obtained. This water-absorbent resin had a median particle size of 368 $\mu$m, absorption capacity of physiological saline solution of 60 g/g, and a water content of 5.1% (A median particle size of primary particles is about 60 $\mu$m).

Example 5

According to the same manner as that of Example 3, except that a solution prepared by warming to dissolve, in 8.28 g of n-heptane by warming, 0.92 g of tetraglyceryl stearate (manufactured by Mitsubishi-Kagaku Foods Corporation, trade name: TS4) in place of 0.92 g of a sucrose fatty acid ester (manufactured by Mitsubishi-Kagaku Foods Corporation, trade name: S-370) was used, and the reaction solution was cooled to about 20°C after completion of the polymerization at the first stage in the step (B) of Example 3, 234 g of a water-absorbent resin having the shape of aggregated spherical particles spherical particles was obtained. This water-absorbent resin had a median particle size of 293 $\mu$m, absorption capacity of physiological saline solution of 58 g/g, and a water content of 3.0% (A median particle size of primary particles of this water-absorbent resin is about 70 $\mu$m).

Example 6

According to the same manner as that of Example 3, except that 0.92 g of an oxidized ethylene-propylene copolymer (manufactured by Mitsui Chemicals, Inc., trade name: HIWAX 4052E) was used as a polymeric dispersion agent in place of 0.92 g of the maleic anhydride-modified ethylene-propylene copolymer (manufactured by Mitsui Chemicals, Inc., trade name: HIWAX 1105A) in the step (A) of Example 3, 240 g of a water-absorbent resin having the shape of aggregated spherical particles spherical particles was obtained. This water-absorbent resin had a median particle size of 353 $\mu$m, absorption capacity of physiological saline solution of 63 g/g, and a water content of 5.8% (A median particle size of primary particles of this water-absorbent resin is about 60 $\mu$m).

Example 7

To a 500 mL Erlenmeyer flask, 92.0 g of 80 mass% acrylic acid was added, and this was neutralized by adding dropwise 102.2 g of 30 mass% sodium hydroxide while cooling the flask from the outside. To this were added 0.11 g of potassium persulfate, 8.3 mg of ethylene glycol diglycidyl ether and 43.6 g of ion-exchange water to prepare an aqueous solution of a water-soluble ethylenically unsaturated monomer.

To a 2 L volumetric five-necked cylindrical round-bottom flask equipped with a stirrer with two 50 mm$\phi$ pitched blade paddle impellers, a thermometer, a reflux condenser and a nitrogen gas introducing tube, 334 g of n-heptane was added as a petroleum hydrocarbon dispersion medium and 0.92 g of a maleic anhydride-modified ethylene-propylene copolymer (manufactured by Mitsui Chemicals, Inc., trade name: HIWAX 1105A) was added as a polymeric dispersion agent and, then, they were dissolved by warming. After cooling to 61°C, the aqueous solution of a water-soluble ethylenically unsaturated monomer was added at once under a stirring rate of 300 rpm using a funnel and then dispersed by stirring at the inner temperature of 40°C for 10 minutes (step (A))

Next, a solution prepared by warming to dissolve 0.92 g of a sucrose fatty acid ester (manufactured by Mitsubishi-Kagaku Foods Corporation, trade name: S-370) as a surfactant in 8.28 g of n-heptane by warming was added to the round-bottom flask using a funnel, and an aqueous solution of a water-soluble ethylenically unsaturated monomer was further dispersed at a stirring rate of 500 rpm (step (B)).

Next, the atmosphere in the system was well substituted with nitrogen while maintaining the inner temperature of the round-bottom flask containing the dispersion at 40°C, and the polymerization reaction was performed by warming for 1 hour using a hot water bath at 70°C (step (C)).

The operation after completion of the polymerization at the first stage was performed according to the same manner as that of Example 3 to obtain 237 g of a water-absorbent resin having the shape of aggregated spherical particles. This water-absorbent resin had a median particle size of 372 $\mu$m, absorption capacity of physiological saline solution of 62 g/g, and a water content of 4.8% (A median particle size of primary particles of this water-absorbent resin is about 60 $\mu$m).

Example 8

According to the same manner as that of Example 7, except that a mixture of 0.46 g of an oxidized ethylene-propylene copolymer (manufactured by Mitsui Chemicals, Inc., trade name: HIWAX 4052E) and 0.46 g of a maleic anhydride-modified ethylene-propylene copolymer (manufactured by Mitsui Chemicals, Inc., trade name: HIWAX 1105A) was used as a polymeric dispersion agent in place of 0.92 g of the maleic anhydride-modified ethylene-propylene copolymer (manufactured by Mitsui Chemicals, Inc., trade name: HIWAX 1105A) in the step (A) of Example 7, 235 g of a water-absorbent resin having the shape of aggregated spherical particles spherical particles was obtained. This water-absorbent resin had a median particle size of 356 $\mu$m, absorption capacity of physiological saline solution of 63 g/g, and a water content of 4.5% (A median particle size of primary particles of this water-absorbent resin is about 60 $\mu$m).

Comparative Example 1

To a 500 mL Erlenmeyer flask, 92.0 g of 80 mass% acrylic acid was added, and this was neutralized by adding dropwise 102.2 g of 30 mass% sodium hydroxide while cooling the flask from the outside. To this were added 0.11 g of potassium persulfate, 8.3 mg of ethylene glycol diglycidyl ether and 43.6 g ion-exchange water to prepare an aqueous solution of a water-soluble ethylenically unsaturated monomer.

To a 2 L volumetric five-necked cylindrical round-bottom flask equipped with a stirrer with two 50 mm$\phi$ pitched blade paddle impellers, a thermometer, a reflux condenser and a nitrogen gas introducing tube, 342 g of n-heptane was added as a petroleum hydrocarbon dispersion medium, and 0.92 g of a sucrose fatty acid ester (manufactured by Mitsubishi-Kagaku Foods Corporation, trade name: S-370) was added as a surfactant, followed by warming to 61°C to dissolve the ester. Thereafter, the aqueous solution of a water-soluble ethylenically unsaturated monomer was added at once using a funnel under a stirring rate of 500 rpm. Next, the atmosphere in the system was well substituted with nitrogen while maintaining the inner temperature at 40°C, and the polymerization reaction was performed by warming for 1 hour using a hot water bath at 70°C.

The operation after completion of the polymerization at the first stage was performed according to the same manner as that of Example 1 to obtain 236 g of a water-absorbent resin having the shape of aggregated spherical particles. This water-absorbent resin had a median particle size of 318 $\mu$m, absorption capacity of physiological saline solution of 62 g/g, and a water content of 4.6% (A median particle size of primary particles of this water-absorbent resin is about 60 $\mu$m).

Comparative Example 2

To a 500 mL Erlenmeyer flask, 92.0 g of 80 mass% acrylic acid was added, and this was neutralized by adding dropwise 102.2 g of 30 mass% sodium hydroxide while cooling the flask from the outside. To this were added 0.11 g of potassium persulfate, 8.3 mg of ethylene glycol diglycidyl ether and 43.6 g ion-exchange water to prepare an aqueous solution of a water-soluble ethylenically unsaturated monomer.

To a 2 L volumetric five-necked cylindrical roundbottom flask equipped with a stirrer with two 50 mm$\phi$ pitched blade paddle impellers, a thermometer, a reflux condenser and a nitrogen gas introducing tube, 342 g of n-heptane was added as a petroleum hydrocarbon dispersion medium, 0.92 g of a sucrose fatty acid ester (manufactured by Mitsubishi-Kagaku Foods Corporation, trade name: S-370) was added as a surfactant, and 0.92 g of a maleic anhydride-modified ethylene-propylene copolymer (manufactured by Mitsui Chemicals, Inc., trade name: HIWAX 1105A) was added as a polymeric dispersion agent, followed by warming to dissolve them. After cooling to 61°C, the aqueous solution of a water-soluble ethylenically unsaturated monomer was added at once using a funnel under a stirring rate of 500 rpm. Next, the atmosphere in the system was well substituted with nitrogen while maintaining the inner temperature at 40°C, and the polymerization reaction was performed by warming for 1 hour using a hot water bath at 70°C.

The operation after completion of the polymerization at the first stage was performed according to the same manner as that of Example 3 to obtain 237 g of a water-absorbent resin having the shape of aggregated spherical particles. This water-absorbent resin had a median particle size of 348 $\mu$m, absorption capacity of physiological saline solution of 61 g/g, and a water content of 4.9% (A median particle of this water-absorbent resin size of primary particles is about 60 $\mu$m).

Comparative Example 3

According to the same manner as that of Comparative Example 2, except that 0.92 g of tetraglyceryl stearate (manufactured by Mitsubishi-Kagaku Foods Corporation, trade name: TS4) was used as a surfactant in place of 0.92 g of the sucrose fatty acid ester (manufactured by Mitsubishi-Kagaku Foods Corporation , trade name: S-370), and the reaction solution was cooled to about 20°C after completion of the polymerization at the first stage in Comparative Example 2, 235 g of a water-absorbent resin having the shape of aggregated spherical particles was obtained. This water-absorbent resin had a median particle size of 273 $\mu$m, absorption capacity of physiological saline solution of 60 g/g, and a water content of 3.5% (A median particle size of primary particles of this water-absorbent resin is about 70 $\mu$m).

Comparative Example 4

The sample obtained according to the same manner as that of Comparative Example 2 was further heated by a hot air dryer at 180°C for 3 hours to obtain 230 g of a water-absorbent resin having the shape of aggregated spherical particles. This water-absorbent resin had a median particle size of 356 $\mu$m, absorption capacity of physiological saline solution of 68 g/g, and a water content of 0.5% (A median particle size of primary particles of this water-absorbent resin is about 60 $\mu$m).

The amount of the remaining dispersion medium and the results of an odor sensory test of water-absorbent resins obtained in Examples 1 to 8 and Comparative Examples 1 to 4 are shown in Table 2.

Table 2

| Samples | Species of dispersion medium | Amount of remaining dispersion medium ppm] | Odor sensory test | | | | | |
|---|---|---|---|---|---|---|---|---|
| | | | Panelist | | | | | |
| | | | A | B | C | D | E | Average |
| Example 1 | Heptane | 1,210 | 2 | 2 | 1 | 1 | 1 | 1.4 |
| Example 2 | Heptane | 960 | 1 | 1 | 1 | 1 | 1 | 1.0 |
| Example 3 | Heptane | 830 | 1 | 1 | 1 | 1 | 1 | 1.0 |
| Example 4 | Heptane | 770 | 1 | 1 | 1 | 1 | 1 | 1.0 |
| Example 5 | Heptane | 590 | 1 | 1 | 1 | 0 | 0 | 0.6 |
| Example 6 | Heptane | 180 | 0 | 0 | 0 | 0 | 0 | 0.0 |
| Example 7 | Heptane | 320 | 0 | 0 | 0 | 0 | 0 | 0.0 |
| Example 8 | Heptane | 80 | 0 | 0 | 0 | 0 | 0 | 0.0 |
| Comparative Example 1 | Heptane | 15,300 | 4 | 4 | 4 | 3 | 3 | 3.6 |
| Comparative Example 2 | Heptane | 5,300 | 3 | 3 | 3 | 3 | 3 | 3.0 |
| Comparative Example 3 | Heptane | 11,100 | 4 | 4 | 3 | 3 | 3 | 3.4 |
| Comparative Example 4 | Heptane | 4,500 | 3 | 3 | 3 | 3 | 3 | 3.0 |

As is apparent from Table 2, in the water-absorbent resin of the present invention, the amount of the petroleum hydrocarbon dispersion medium remaining used during the polymerization of the water-absorbent resin is remarkably decreased to 2,000 ppm or less and an odor originating from the dispersion medium is reduced. In the water-absorbent resins of Examples 6 to 8 having the amount of the remaining dispersion medium of 500 ppm or less, no odor originating from the dispersion medium was felt in the odor sensory test.

Industrial Applicability

According to the present invention, there are provided a water-absorbent resin which contains a small remaining amount of a petroleum hydrocarbon dispersion medium used in reversed-phase suspension polymerization, and thus reducing an odor originating from the petroleum hydrocarbon dispersion medium, and also which is suitable for use in hygienic materials; an absorbent material and an absorbent article using the same.

**Claims**

1. A method for producing a water-absorbent resin comprising performing a reversed-phase suspension polymerization at multi-stages of two or more stages, and performing the reversed-phase suspension polymerization at the first stage by:

   (A) adding an aqueous solution of a water-soluble ethylenically unsaturated monomer to a petroleum hydrocarbon dispersion medium to disperse the aqueous solution in the dispersion medium,
   (B) adding a surfactant to the resultant dispersion to further disperse the aqueous solution, and
   (C) performing the reversed-phase suspension polymerization at the first stage using a water-soluble radical polymerization initiator, optionally in the presence of an internal-crosslinking agent.

2. The method for producing a water-absorbent resin according to claim 1, wherein the aqueous solution of a water-soluble ethylenically unsaturated monomer is added to the petroleum hydrocarbon dispersion medium in the presence of a polymeric dispersion agent to disperse the aqueous solution in the dispersion medium in the step (A).

3. The method according to claim 1 or 2, wherein a polymeric dispersion agent is added together with the surfactant in the step (B).

4. The method according to any one of claims 1 to 3, further comprising adding a post-crosslinking agent to a precursor of the water-absorbent resin obtained after completion of the multi-stage reversed-phase suspension polymerization including the steps (A) to (C), and post-crosslinking the precursor.

5. The method according to any one of claims 1 to 4, wherein the surfactant is at least one kind selected from the group consisting of a polyglycerin fatty acid ester, a sucrose fatty acid ester and a sorbitan fatty acid ester.

6. The method according to any one of claims 2 to 5, wherein the polymeric dispersion agent is at least one kind selected from the group consisting of maleic anhydride-modified polyethylene, maleic anhydride-modified polypropylene, a maleic anhydride-modified ethylene-propylene copolymer, a maleic anhydride-ethylene copolymer, a maleic anhydride-propylene copolymer, a maleic anhydride-ethylene-propylene copolymer, polyethylene, polypropylene, an ethylene-propylene copolymer, oxidized polyethylene, oxidized polypropylene and an oxidized ethylene-propylene copolymer.

7. The method according to any one of claims 1 to 6, wherein the water-soluble ethylenically unsaturated monomer is at least one kind selected from the group consisting of acrylic acid and a salt thereof, methacrylic acid and a salt thereof, and acrylamide.

8. The method according to any one of claims 1 to 7, wherein the petroleum hydrocarbon dispersion medium is at least one kind selected from the group consisting of an aliphatic hydrocarbon and an alicyclic hydrocarbon, each having 6 to 7 carbon atoms.

9. A method according to any one of the preceding claims wherein the multi-stage reversed-phase suspension polymerisation includes a second stage in which an aqueous solution of the water-soluble ethylenically unsaturated monomer is added to the polymerization reaction suspension produced in the first stage, and reversed-phase polymerization is carried out thereby to produce aggregate particles of the water-absorbent resin.

**Patentansprüche**

1. Ein Verfahren zur Herstellung eines wasserabsorbierenden Harzes, umfassend Durchführen einer mehrstufigen Umkehrphasen-Suspensionspolymerisation mit zwei oder mehr Stufen und Durchführen der Umkehrphasen-Suspensionspolymerisation in der ersten Stufe durch:

   (A) Zugabe einer wässrigen Lösung eines wasserlöslichen ethylenisch ungesättigten Monomers zu einem Erdölkohlenwasserstoff-Dispersionsmedium, um die wässrige Lösung in dem Dispersionsmedium zu dispergieren,
   (B) Zugabe eines grenzflächenaktiven Mittels zu der resultierenden Dispersion, um die wässrige Lösung weiter zu dispergieren, und

(C) Durchführen der Umkehrphasen-Suspensionspolymerisation in der ersten Stufe unter Verwendung eines wasserlöslichen radikalischen Polymerisationsinitiators, gegebenenfalls in Gegenwart eines internen Vernetzungsmittels.

**2.** Das Verfahren zur Herstellung eines wasserabsorbierenden Harzes nach Anspruch 1, wobei die wässrige Lösung eines wasserlöslichen ethylenisch ungesättigten Monomers dem Erdölkohlenwasserstoff-Dispersionsmedium in Gegenwart eines polymeren Dispergiermittels zugegeben wird, um die wässrige Lösung in dem Dispersionsmedium in Schritt (A) zu dispergieren.

**3.** Das Verfahren nach Anspruch 1 oder 2, wobei ein polymeres Dispergiermittel zusammen mit dem grenzflächenaktiven Mittel in Schritt (B) zugegeben wird.

**4.** Das Verfahren nach einem der Ansprüche 1 bis 3, weiter umfassend Zugabe eines Nachvernetzungsmittels zu einer Vorstufe des nach Beendigung der mehrstufigen, die Schritte (A) bis (C) beinhaltenden Umkehrphasen-Suspensionspolymerisation erhaltenen wasserabsorbierenden Harzes und Nachvernetzen der Vorstufe.

**5.** Das Verfahren nach einem der Ansprüche 1 bis 4, wobei das grenzflächenaktive Mittel mindestens eine Art, ausgewählt aus der Gruppe bestehend aus einem Polyglycerolfettsäureester, einem Saccharosefettsäureester und einem Sorbitanfettsäureester, ist.

**6.** Das Verfahren nach einem der Ansprüche 2 bis 5, wobei das polymere Dispergiermittel mindestens eine Art, ausgewählt aus der Gruppe bestehend aus Maleinsäureanhydrid-modifiziertem Polyethylen, Maleinsäureanhydrid-modifiziertem Polypropylen, einem Maleinsäureanhydrid-modifizierten Ethylen-Propylen-Copolymer, einem Maleinsäureanhydrid-Ethylen-Copolymer, einem Maleinsäureanhydrid-Propylen-Copolymer, einem Maleinsäureanhydrid-Ethylen-Propylen-Copolymer, Polyethylen, Polypropylen, einem Ethylen-Propylen-Copolymer, oxidiertem Polyethylen, oxidiertem Polypropylen und einem oxidierten Ethylen-Propylen-Copolymer, ist.

**7.** Das Verfahren nach einem der Ansprüche 1 bis 6, wobei das wasserlösliche ethylenisch ungesättigte Monomer mindestens eine Art, ausgewählt aus der Gruppe bestehend aus Acrylsäure und einem Salz davon, Methacrylsäure und einem Salz davon und Acrylamid, ist.

**8.** Das Verfahren nach einem der Ansprüche 1 bis 7, wobei das Erdölkohlenwasserstoff-Dispersionsmedium mindestens eine Art, ausgewählt aus der Gruppe bestehend aus einem aliphatischen Kohlenwasserstoff und einem alicyclischen Kohlenwasserstoff, jeweils mit 6 bis 7 Kohlenstoffatomen, ist.

**9.** Ein Verfahren nach einem der vorhergehenden Ansprüche, wobei die mehrstufige Umkehrphasen-Suspensionspolymerisation eine zweite Stufe beinhaltet, in der eine wässrige Lösung des wasserlöslichen ethylenisch ungesättigten Monomers der in der ersten Stufe erhaltenen Polymerisationsreaktionssuspension zugegeben wird und Umkehrphasenpolymerisation durchgeführt wird, wodurch Aggregatteilchen des wasserabsorbierenden Harzes erhalten werden.

## Revendications

**1.** Procédé de production d'une résine absorbant l'eau comprenant la réalisation d'une polymérisation en suspension en phase inversée en plusieurs stades de deux stades ou plus, et la réalisation de la polymérisation en suspension en phase inversée au premier stade par :

(A) ajout d'une solution aqueuse d'un monomère à insaturation éthylénique hydrosoluble à un milieu de dispersion d'hydrocarbure de pétrole pour disperser la solution aqueuse dans le milieu de dispersion,
(B) ajout d'un tensioactif à la dispersion résultante pour davantage disperser la solution aqueuse, et
(C) réalisation de la polymérisation en suspension en phase inversée au premier stade en utilisant un initiateur de polymérisation radicalaire hydrosoluble, éventuellement en présence d'un agent de réticulation interne.

**2.** Procédé de production d'une résine absorbant l'eau selon la revendication 1, dans lequel la solution aqueuse d'un monomère à insaturation éthylénique hydrosoluble est ajoutée au milieu de dispersion d'hydrocarbure de pétrole en présence d'un agent de dispersion polymère pour disperser la solution aqueuse dans le milieu de dispersion dans l'étape (A).

**3.** Procédé selon la revendication 1 ou 2, dans lequel un agent de dispersion polymère est ajouté avec le tensioactif de l'étape (B).

**4.** Procédé selon l'une quelconque des revendications 1 à 3, comprenant en outre l'ajout d'un agent de post-réticulation à un précurseur de la résine absorbant l'eau obtenue après l'achèvement de la polymérisation en suspension en phase inversée en plusieurs stades comprenant les étapes (A) à (C) et la post-réticulation du précurseur.

**5.** Procédé selon l'une quelconque des revendications 1 à 4, dans lequel le tensioactif est au moins un type choisi dans le groupe constitué par un ester d'acide gras de polyglycérine, un ester d'acide gras de saccharose et un ester d'acide gras de sorbitane.

**6.** Procédé selon l'une quelconque des revendications 2 à 5, dans lequel l'agent de dispersion polymère est au moins un type choisi dans le groupe constitué par un polyéthylène modifié par de l'anhydride maléique, un polypropylène modifié par de l'anhydride maléique, un copolymère d'éthylène-propylène modifié par de l'anhydride maléique, un copolymère d'anhydride maléique-éthylène, un copolymère d'anhydride maléique-propylène, un copolymère d'anhydride maléique-éthylène-propylène, un polyéthylène, un polypropylène, un copolymère d'éthylène-propylène, un polyéthylène oxydé, un polypropylène oxydé et un copolymère d'éthylène-propylène oxydé.

**7.** Procédé selon l'une quelconque des revendications 1 à 6, dans lequel le monomère à insaturation éthylénique hydrosoluble est au moins un type choisi dans le groupe constitué par l'acide acrylique et un sel de celui-ci, l'acide méthacrylique et un sel de celui-ci, et l'acrylamide.

**8.** Procédé selon l'une quelconque des revendications 1 à 7, dans lequel le milieu de dispersion d'hydrocarbure de pétrole est au moins un type choisi dans le groupe constitué par un hydrocarbure aliphatique et un hydrocarbure alicyclique, chacun ayant de 6 à 7 atomes de carbone.

**9.** Procédé selon l'une quelconque des revendications précédentes, dans lequel la polymérisation en suspension en phase inversée en plusieurs stades comprend une seconde phase dans laquelle une solution aqueuse du monomère à insaturation éthylénique hydrosoluble est ajoutée à la suspension de réaction de polymérisation produite dans la première phase, et la polymérisation en phase inversée est réalisé ensuite pour produire des particules agrégées de la résine absorbant l'eau.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2014683 A1
- JP 2006068731 A
- JP 3195713 A
- JP 3195709 A
- JP 61087702 A
- JP 62172006 A